# EUROPÄISCHE PATENTSCHRIFT

(11) **EP 3 694 645 B1**
(45) Veröffentlichungstag und Bekanntmachung des Hinweises auf die Patenterteilung: **01.12.2021**
(21) Anmeldenummer: 18793575.4
(22) Anmeldetag: 10.10.2018
(51) Int. Cl.: B01L 3/00, A61M 1/02, A61M 1/36

(54) **VORRICHTUNG MIT EINER ERSTEN UND EINER ZWEITEN KAMMER ZUR AUFNAHME EINES KÖRPERFLUIDS**
DEVICE HAVING A FIRST AND A SECOND CHAMBER FOR RECEIVING A BODY FLUID
DISPOSITIF COMPORTANT UNE PREMIÈRE ET UNE DEUXIÈME CHAMBRE POUR LA RÉCEPTION D'UN FLUIDE CORPOREL

(30) Priorität: 11.10.2017 EP 17195982
(43) Veröffentlichungstag der Anmeldung: 19.08.2020
(73) Patentinhaber: Orthogen AG, 40212 Düsseldorf (DE)
(72) Erfinder: BREIDENBACH, Nina, 40231 Düsseldorf (DE); REINECKE, Julio, 40733 Köln (DE); TROILLET, Julien, 04416 Markkleeberg (DE); WEHLING, Peter, 40597 Düsseldorf (DE); HEINDIRK, Julia, 41352 Korschenbroich (DE)
(74) Vertreter: König, Gregor Sebastian
(86) Internationale Anmeldenummer: PCT/EP2018/077574
(87) Internationale Veröffentlichungsnummer: WO 2019/072903

(56) Entgegenhaltungen:
- US-A- 3 706 305
- US-A- 4 209 488
- US-A- 4 644 807
- US-A- 5 360 011
- US-A1- 2008 166 421

## Beschreibung

Die vorliegende Erfindung betrifft eine Vorrichtung und ein Verfahren zum Überführen von Körperflüssigkeiten, sowie eine Verwendung.

Vorrichtungen für die Separation einzelner Blutphasen, beispielsweise mittels der Anwendung einer Zentrifuge, oder für die Herstellung und Abtrennung biologisch aktiver Substanzen, wie beispielsweise autologen Proteinen, sind bekannt.

US 2008/0166421 A1 offenbart ein Verfahren zur Prozessierung eines Plasmas, bei dem eine spritzenähnliche Vorrichtung als Kolbenbestandteil einer weiteren spritzen-ähnlichen Vorrichtung ausgestaltet ist. Es sind demnach zwei spritzenähnliche Vorrichtungen "hintereinandergeschaltet". Damit werden zwei Kammern verwendet, die jede eine verschiebliche Wandung aufweisen. Hierdurch wird eine Gesamtvorrichtung geschaffen, die einen geübten Anwender erfordert. Die Handhabungsschritte sind im Hinblick auf die gegeneinander und vom Anwender zu bedienenden bzw. bewegenden Teile komplex. Eine Automatisierung ist nicht einfach möglich.

US 4,644,807 A offenbart eine Vorrichtung zur Überführung von Proben einer Flüssigkeit, die in einer Chromatographiesäule analysiert werden sollen. Es wird ein Probenbehältnis beschrieben, in dem ein Stempel gleitverschieblich bewegt werden kann. Durch das Verschieben des Stempels kann die in dem Probenbehältnis vorhandene Flüssigkeit zusammengedrückt werden und steigt unter Verdrängung von Luft in einer Säule auf. Eine simple, insbesondere sterile, Prozessierung der Flüssigkeit ist nicht gegeben.

US 4,209,488 A offenbart eine Vorrichtung zur Probenprozessierung. Es sind zwei voneinander getrennte Kammern vorgesehen.

EP 2 123 289 A1 offenbart eine Vorrichtung zur Herstellung einer biologisch aktiven Substanz. Es wird ein flügelfrei und kolbenlos ausgestalteter Container beschrieben, dem Blutbestandteile nach einer Blutentnahme und/oder einer Aufbereitung zugeführt werden können. Der Container enthält Perlen als Scherkörper, die dazu dienen, die Blutbestandteile einer Stresssituation auszusetzen, was zur Bildung einer biologisch aktiven Substanz führen kann. Nach Herstellung der biologisch aktiven Substanz kann der Container in eine Zentrifuge überführt werden und Blutphasen entsprechend ihrer Dichte voneinander getrennt werden. Neben dem Container ist ein weiteres Behältnis für die Entnahme bzw. Aufbereitung des Blutes sowie ein Transfer zwischen dem separaten Behältnis und dem Container notwendig. Dies führt zu vielen Verfahrensschritten, die ein Anwender ausführen muss, wobei gleichzeitig die Gefahr von Kontaminationen besteht.

DE 603 14 413 T2 beschreibt ein Verfahren sowie eine Vorrichtung zur Isolierung von Plättchen aus Blut, wobei einer Abtrennvorrichtung für Blutplättchen das Blut mittels einer externen Blutentnahmevorrichtung in einem vorgelagerten Schritt zugeführt wird. Der Transfer des Blutes in verschiedene Behältnisse birgt das Risiko von Kontaminationen und erfordert mehrere Verfahrensschritte.

US 4,828,716 A offenbart eine Vorrichtung sowie ein Verfahren zur Separation von Blutphasen. Die Vorrichtung umfasst eine röhrenförmige Kammer, die evakuiert wurde und in der ein Unterdruck vorliegt. Mittels Verwendung einer doppelendigen Nadel kann eine direkt Blutbefüllung durch den Unterdruck während einer Blutentnahme stattfinden. Die mit Blut gefüllte Kammer kann um ihre Longitudinalachse rotiert werden, um die einzelnen Phasen des Blutes voneinander zu trennen, so dass die Phasen konzentrisch zueinander in der Kammer vorliegen. Um die Phasen weiter voneinander zu trennen wird die Kammer um ihre Longitudinalachse rotiert und ein Anwender schiebt mit einem Stab ein Trennelement in die Kammer, so dass sich die mittig angeordnete Phase in der Kammer nach oben verlagert, wobei ein Druckausgleich durchgeführt wird. Die Verfahrensschritte sind aufwendig und bergen das Risiko einer Kontamination.

US 8,052,969 B2 beschreibt eine Methode zur Herstellung von mit Plättchen angereichertem Blutplasma unter Verwendung einer Zwei-Kammer-Spritze. Bei der Zwei-Kammer-Spritze ist der Kolben einer ersten Spritze wiederum als eine (zweite) Spritze ausgestaltet. Nach einer Blutbefüllung der ersten Spritze kann die Zwei-Kammer-Spritze einem Zentrifugationsprozess unterzogen werden, wobei sich die einzelnen Bestandteile in Abhängigkeit ihrer Dichte voneinander trennen. In einem der Zentrifugation nachgelagerten Schritt kann mittels der zweiten Spritze die obere, Plättchen angereicherte Plasmaschicht von der unteren abgetrennt werden. Hierbei wird das Plasma in den Hohlraum der zweiten Spritze aufgezogen. Danach kann die zweite Spritze von der ersten Spritze getrennt werden. Die durchzuführenden Verfahrensschritte erfordern einen geübten Anwender, wobei Kontaminationen nicht ausgeschlossen werden können.

US 3,706,305 A beschreibt eine Vorrichtung, die eine Kombination aus Vakuumspritze für die Blutentnahme, Zentrifugencontainer und Probenbehältnis darstellt. Es werden Kammern mit konstanter effektiver Größe miteinander kombiniert. Die für die Aufnahme des Bluts vorgesehenen Kammern haben eine konstante effektive Größe. Dies führt zu einem relativ langen Aufbau, wobei zudem eine relativ unflexible Handhabungsmöglichkeit geschaffen wird. Nach Blutentnahme wird das Blut in eine Zentrifugationskammer geleitet. Anschließend können die einzelnen Blutphasen durch Zentrifugation voneinander getrennt werden. Um die Serumphase in einem nachgelagertem Schritt von den restlichen Blutbestandteilen zu trennen und in das unter Vakuum stehende Probenbehältnis zu transferieren, muss dieses manuell über eine Transfernadel mit dem Inneren der Zentrifugationskammer in Verbindung gebracht werden. Atmosphärische Luft strömt hierbei für einen Druckausgleich in die Zentrifugationskammer. Die durchzuführenden Verfahrensschritte erfordern einen geübten Anwender, wobei eine Kontamination der Blutbestandteile nicht ausgeschlossen werden kann.

US 6,398,972 B1 offenbart ein Verfahren zum Erzeugen eines mit Plättchen angereicherten Plasmas, bei dem eine Vorrichtung verwendet wird, bei der zwei einstückig ausgebildete Behältnisse vorhanden sind. Die zwei Behältnisse sind nebeneinander angeordnet und mittels eines Strömungskanals ständig miteinander verbunden. Zum Überführen von Phasen wird ein Anwender benötigt oder eine von der Zentrifugation zur Separierung der Phasen unterschiedliche Behandlung in der Zentrifuge.

US 5,360,011 A offenbart eine Blutentnahme, bei der in einem ersten Schritt Blut mittels einer Spritze entnommen wird, um zu gewährleisten, dass eine Blutentnahme an der Stelle, an der die Nadel der Spritze eingestochen wurde, möglich ist. Sofern mit der Spritze eine geeignete Stelle zur Blutentnahme gefunden wurde, wird ein unter Unterdruck stehendes Gefäß an die Spritze herangeführt. Das unter Unterdruck stehende Gefäß wird dann mit dem Blut aus der Spritze und nachfließendem Blut befüllt, ohne dass der Kolben der Spritze verschoben wird. Eine durchgehend sterile Überführung ist nicht beschrieben. Das aus der US 5,360,011 A bekannte Verfahren erfordert auch einen geübten Anwender.

Vor diesem Hintergrund ist es eine Aufgabe der Erfindung, eine Vorrichtung und ein Verfahren sowie eine Verwendung bereitzustellen, die bzw. das einen Transfer von einer Phase einer Körperflüssigkeit verbessert, wobei eine Verbesserung darin gesehen werden kann, eine möglichst geringe Anzahl von Verfahrensschritten eines Anwenders zu ermöglichen, die Handhabung zu verbessern und/oder einen Transfer des Körperfluids möglichst steril durchzuführen.

Die Aufgabe wird gelöst durch den Gegenstand des unabhängigen Patentanspruchs. Vorteilhafte Ausführungsformen sind Gegenstand der abhängigen Patentansprüche und ergeben sich aus der nachfolgenden Beschreibung.

Es wird ein erster Aspekt mit einer Vorrichtung zur Aufnahme eines Körperfluids beschrieben, wobei die Vorrichtung eine erste Kammer, die eine verschiebliche Wandung aufweist, und eine zweite Kammer, mit konstanter effektiver Größe, die in Bezug auf die verschiebliche Wandung bewegbar ist, umfasst, wobei die Vorrichtung ausgestaltet ist, bei einer längsaxialen Bewegung der zweiten Kammer in Bezug auf die verschiebliche Wandung die erste Kammer und die zweite Kammer zu verbinden und bei einer Bewegung der zweiten Kammer zusammen mit der verschieblichen Wandung Körperfluid von der ersten Kammer in die zweite Kammer zu überführen. Hierdurch kann eine einfach ausgestaltete und einfach handhabbare Vorrichtung geschaffen werden, bei der eine längsaxiale Bewegung die Überführung bewirken kann. Ferner wird ein zweiter Aspekt beschrieben, gemäß dem der strukturelle Aufbau der Vorrichtung gemäß dem ersten Aspekt verwendet werden kann, wobei eine Vorrichtung mit einer ersten Kammer zur Aufnahme eines Körperfluids und einer zweiten Kammer beschrieben wird. Bei der Zentrifugation zur Separation der Phasen des Körperfluids kann die erste und die zweite Kammer verbunden werden und Körperfluid bei der Zentrifugation mittels der Zentrifugalkraft aus der ersten Kammer in die zweite Kammer überführt werden Die Anzahl der Verfahrensschritte eines Anwenders wird reduziert. Gemäß dem zweiten Aspekt kann vorgesehen sein, dass die erste Kammer keine verschiebliche Wandung aufweist, sondern als ein Behältnis ausgeführt ist, welches ein im Wesentlichen konstantes Volumen und einen Unterdruck aufweist.

Es wird auch ein dritter Aspekt einer Vorrichtung beschrieben, gemäß dem der strukturelle Aufbau wie die Vorrichtung gemäß dem ersten Aspekt beschrieben ist, wobei die Vorrichtung eine erste Kammer zur Aufnahme eines Körperfluids und eine zweite Kammer aufweist. Die Vorrichtung ist ausgestaltet - insbesondere nach einer Zentrifugation und einer Separation der Phasen des Körperfluids - eine längsverschiebliche Bewegung auszuführen, bei der die Vorrichtung zusammengedrückt und Körperfluid von der ersten Kammer in die zweite Kammer überführt wird. Hierdurch kann eine einfach ausgestaltete und einfach handhabbare Vorrichtung beschrieben werden.

Es wird der Grundgedanke beschrieben, bei einer Zentrifugation die wirkenden Kräfte auszunutzen, um eine durch die Kräfte induzierte Bewegung zu ermöglichen, bei der Kammern miteinander verbunden werden können und ein Transfer bzw. eine Überführung des Körperfluids bei der Zentrifugation aufgrund der wirkenden Kräfte zumindest teilweise zwischen den Kammern ermöglicht wird. Es wurde erkannt, dass für die Separation bzw. Trennung der einzelnen Phasen bzw. Komponenten von Körperfluiden Kräfte aufgebracht werden, die neben der Separation bzw. Trennung der einzelnen Phasen für einen Transfer einer Phase von einer Kammer in eine andere Kammer genutzt werden können.

Der erste Aspekt kann mit dem zweiten Aspekt sowie der erste Aspekt mit dem dritten Aspekt kombiniert werden. Es kann auch vorgesehen sein, dass der zweite und der dritte Aspekt miteinander kombiniert werden können, um bspw. eine zumindest teilweise Überführung während einer Zentrifugation und im Anschluss an die Zentrifugation eine weitere oder abschließende Überführung von Körperfluid zu ermöglichen.

Es kann eine Vorrichtung bereitgestellt werden, die eine Entnahme, eine Modifizierung, eine Separation, eine Isolierung und/oder eine Lagerung von Körperfluidkomponenten, insbesondere von Phasen, die mittels einer Zentrifugation voneinander separiert werden können, mittels einer einzigen Vorrichtung ermöglicht. Hierdurch kann eine möglichst sterile Handhabung von Körperfluiden gewährleistet werden. Der Anwender muss keine Schritte mehr durchführen, die großer Übung und/oder Erfahrung bedürfen.

Der Begriff "Körperfluid" im Sinne der Beschreibung umfasst ein Fluid des menschlichen oder tierischen Körpers.. Der Begriff umfasst im Wesentlichen eine Flüssigkeit, wobei gasförmige oder feste Anteile nicht ausgeschlossen sein sollen. Der Begriff Körperfluid umfasst insbesondere Speichel, Lymphflüssigkeit, Urin, Knochenmark und bevorzugt Blut. Der Begriff "Phase" oder "Komponente" des Körperfluids umfasst Anteile, die mittels einer Zentrifugation voneinander getrennt werden können.

Der Begriff "Kammer" im Sinne der Beschreibung umfasst einen Hohlraum, der das Körperfluid und/oder einzelne oder mehrere Phasen des Körperfluids aufnehmen kann. Die Wandungen des Hohlraums können bevorzugt starr ausgebildet sein. Insbesondere können Seitenwandungen der Kammer, d.h. Wandungen, die sich längs der Längsachse der Kammer strecken, starr ausgebildet sein. Die effektive Größe des Hohlraums kann je nach Ausführungsform konstant (zweite Kammer) oder variabel (erste Kammer) sein, wobei der Hohlraum eine maximale Größe haben kann und durch Bewegen einer Wandung, die sich insbesondere in einer Richtung quer zur Längsachse der Kammer erstrecken kann, die Größe verringert oder vergrößert werden kann, um beispielsweise das enthaltene Fluid aus der Kammer (der ersten Kammer) zu drücken oder in die Kammer (erste Kammer) hineinzusaugen. Im Sinne der vorliegenden Beschreibung ist die zweite Kammer gegenüber der Umgebung geschlossen, wobei eine Kammer insbesondere nach Ausbildung einer sterilen Verbindung zum menschlichen oder tierischen Körper geöffnet werden kann, um die Körperflüssigkeit aufzunehmen oder abzugeben. In einer bevorzugten Ausführungsform kann eine der beiden Kammern aus der Vorrichtung entfernt werden. Eine der Kammern kann derart eingerichtet und angeordnet sein, um von der Vorrichtung getrennt zu werden, wobei der Inhalt der Kammer steril in der Kammer verbleiben kann. Die Kammern können derart ausgestaltet sein, dass diese voneinander separiert werden können, und zumindest eine Kammer kann geschlossen verbleiben oder nach der Separation geschlossen sein. Insbesondere kann die zu entnehmende Kammer in einem Anfangszustand bzw. Auslieferungszustand der Vorrichtung steril verschlossen sein. Alternativ oder zusätzlich ist auch die andere Kammer in diesem Zustand steril verschlossen. Die Kammern können in diesem Zustand geschlossene Einheiten bilden. Eine mögliche Verbindung bzw. Verbindungselemente, die die Verbindung der Kammern ermöglichen, können steril, insbesondere in einem Zwischenraum zwischen den Kammern, vorliegen.

Eine Kammer konstanter effektiver Größe ist eine Kammer, bei der das Gesamtvolumen, das in die Kammer eingefüllt werden kann, gleich bzw. konstant bleibt. Die Wandungen der Kammer mit konstanter effektiver Größe können als unverschieblich zueinander ausgestaltet sein. Insoweit kann der Ausdruck "Kammer mit konstanter effektiver Größe" gleichbedeutend mit "Kammer mit unverschieblichen Wandungen" sein. Dabei ist nicht ausgeschlossen, dass sich die Wandungen der Kammer mit konstanter effektiver Größe, insbesondere geringfügig, wölben oder entfalten können. Eine Kammer konstanter effektiver Größe kann auch ein innerhalb eines faltenbalgähnlichen Konstrukts, das endseitig zumindest an einer Seite geschlossen ist, ausgebildeter Hohlraum sein, der mit Fluid gefüllt werden kann und sich bei der Füllung zumindest teilweise entfaltet. Anstoßabschnitte zwischen den Wandungen der Kammer mit konstanter effektiver Größe können fest ausgeführt sein. Die Wandungen können fest miteinander verbunden sein. Bevorzugt weist die Kammer mit konstanter effektiver Größe einen Unterdruck auf.

Die Vorrichtung kann daher derart eingerichtet und ausgestaltet sein, um bei Zentrifugation zur Separation von Phasen des Körperfluids die erste und die zweite Kammer zu verbinden und das Körperfluid bei dieser Zentrifugation mittels der dabei wirkenden Kräfte aus der ersten Kammer in die zweite Kammer zu überführen Ebenso können die erste Kammer, die zweite Kammer und/oder nachfolgend beschriebene Elemente der Vorrichtung eingerichtet, angeordnet oder ausgestaltet sein, diese Funktion zu ermöglichen oder zu unterstützen.

In einer erfindungsgemäßen Ausführungsform sind die beiden Kammern der Vorrichtungen nach Aufnahme des Körperfluids in der ersten Kammer gegenüber der Umgebung (weiterhin) abgeschlossen. Die Vorrichtung kann insbesondere steril ausgeliefert werden. Dann kann die Vorrichtung steril mit Körperfluid befüllt werden. Im Folgenden kann das Körperfluid steril in der Vorrichtung prozessiert werden. Die Sterilität kann durch den gesamten Prozess, insbesondere der Blutbefüllung, Separation der Phasen und Überführung einer Phase in die zweite Kammer, aufrechterhalten werden bzw. gewährleistet sein.

Insbesondere kann die erste Kammer für eine Körperfluidentnahme, insbesondere eine Blutentnahme, aus dem Körper eingerichtet oder ausgestaltet sein. An der Kammer kann eine, insbesondere wiederverschließbare, insbesondere bevorzugt automatisch bzw. selbsttätig wiederverschließbare, Öffnung oder ein Verbindungsteil ausgebildet sein, mittels der bzw. dem eine Körperfluidentnahme mittels der Vorrichtung durchführbar ist. Vorzugsweise ist ein Septum als Verschlussmembran an einer Öffnung der ersten Kammer derart angeordnet, dass ein, insbesondere automatische bzw. selbsttätige, Wiederverschließen nach einer Befüllung möglich ist. Hierzu kann endseitig an der ersten Kammer, insbesondere an einem sich verengenden Bereich, eine Öffnung mit einem Septum angeordnet sein. Das Septum kann zum Befüllen der Kammer durchstochen und nach dem Befüllen wieder geschlossen werden. Die erste Kammer kann eine endseitige Wandung aufweisen, die verschieblich an der Seitenwandung bewegt werden kann, um das effektive Volumen der Kammer zu verändern. Beispielsweise kann die erste Kammer endseitig eine mittels eines Stempels oder eines Kolbens bewegbare Wandung aufweisen. Die erste Kammer kann einen "zylindrischen Abschnitt" aufweisen bzw. als solche ausgebildet sein, der einen röhrenförmigen Abschnitt aufweist, der von einer Mantelfläche und zwei weiteren Querflächen, die im Wesentlichen Schnittflächen der Mantelfläche sein können, eingeschlossen sein kann. An einer der Querflächen kann die Öffnung mit dem Septum ausgebildet sein und die andere Querfläche kann die verschiebliche Wandung sein. Die Querfläche mit der Öffnung kann einstückig mit der Seitenwandung ausgebildet sein. Der "zylindrische Abschnitt" kann als eine ebene Kurve in einer Ebene dargestellt werden, die entlang einer Geraden, die nicht in der zuvor genannten Ebene enthalten ist, um eine vorgegebene Strecke verschoben wird. Die ebene Kurve kann an der Anfangs- und Endlage der Verschiebung eine oder beide der zuvor genannten Querflächen sein. Die ebene Kurve kann insbesondere ein Kreis, eine Ellipse, ein Polygon oder eine Kombination der zuvor genannten Formen sein. Besonders bevorzugt ist als zylindrischer Abschnitt in dem Sinne der Beschreibung ein senkrechter Kreiszylinder. Die verschiebliche Wandung weist im Falle eines senkrechten Kreiszylinders eine im Querschnitt im Wesentlichen kreisförmige Form auf. Die verschiebliche Wandung der Kammer kann insbesondere als Endfläche eines in einem Zylinder verschieblichen Kolbens vorliegen. Die Öffnung der ersten Kammer mit dem Verbindungsteil und/oder dem Septum kann an der Wand ausgebildet sein, die als Querfläche der verschieblichen Wandung gegenüberliegt. Es kann vorgesehen sein, dass die Befüllung der ersten Kammer mittels eines Anschlusses einer Nadel oder Kanüle am Verbindungsteil und durch eine Bewegung der verschieblichen Wandung der ersten Kammer erfolgt, wobei die Bewegung der verschieblichen Wandung der ersten Kammer in einer besonders bevorzugten Ausführungsform mittels eines Anschlagelements begrenzt ist. Vorzugsweise ist das Füllvolumen der ersten Kammer durch die verschiebbare Wandung variabel. Das Füllvolumen der ersten Kammer kann 2 bis 500 ml betragen. Es ist möglich, dass das Füllvolumen der ersten Kammer 5 bis 500 ml, bevorzugt 5 bis 300 ml, ferner bevorzugt 5 bis 150 ml, weiter bevorzugt 5 bis 100 ml, vorzugsweise 5 bis 50 ml, besonders bevorzugt 10 bis 15 ml, beträgt. Es kann auch vorgesehen sein, dass die erste Kammer ein Vakuum aufweist und eine Befüllung nach dem Vakuumprinzip erfolgen kann. Im Falle, dass die erste Kammer ein Vakuum aufweist, herrscht in der ersten Kammer ein Unterdruck, so dass zur Befüllung der ersten Kammer keine verschiebliche Wandung benötigt wird.

Ein Septum im Sinne der Beschreibung umfasst einen sich üblicherweise von selbst verschließenden Verschluss, insbesondere eine elastomere Dichtung und/oder Verschlussmembran. Es kann aber auch vorgesehen sein, dass ein Septum im Sinne der Beschreibung einen Verschluss beschreibt, der von außen betätigt werden muss, damit der Verschluss wieder verschließt, beispielweise ein Schieber. Im Sinne der Beschreibung kann ein Septum auch ein selbstschließendes Ventil oder eine funktionsgleiche Struktur eines Verschlusses sein, der derart ausgestaltet ist, nach Einwirken (beispielsweise durch Druck und/oder eine Nadel) selbsttätig wieder zu verschließen.

Der Begriff "Kolben" im Sinne der Beschreibung umfasst ein bewegliches Bauteil, das zusammen mit einem Gehäuse, insbesondere dem zylindrischen Abschnitt, einen abgeschlossenen Hohlraum (erste Kammer) bilden kann, dessen Volumen sich durch die Bewegung des Kolbens verändert. Der Kolben kann auf eine Platte oder Scheibe (verschiebliche Wandung), die in dem zylindrischen Abschnitt bewegt wird, reduziert sein.

In einer bevorzugten Ausführungsform weist die Vorrichtung eine Barriere zwischen der ersten Kammer und der zweiten Kammer auf, wobei die Barriere bei einer Befüllung der ersten Kammer vorzugsweise geschlossen ist. Die Barriere kann wahlweise einen Durchfluss von Fluid verhindern oder ermöglichen. Die Barriere kann insbesondere an der ersten Kammer angeordnet sein und eine oder mehrere Öffnungen der ersten Kammer, die einen Fluss zumindest eines Teils des Körperfluids in Richtung der zweiten Kammer ermöglichen, verschließen. Die Barriere und die eine oder die mehreren Öffnungen können an der der Öffnung für eine Körperfluidbefüllung gegenüberliegenden Wandung der Kammer angeordnet sein. Die Barriere kann Teil der ersten Kammer sein, so dass die Barriere zusammen mit der ersten Kammer gehandhabt und/oder bewegt werden kann.

In einer bevorzugten Ausführungsform kann das Körperfluid nach Entnahme bzw. Befüllen in die erste Kammer innerhalb der ersten Kammer modifiziert werden. Hierzu können Additive in der ersten Kammer vorgesehen sein. Typische Additive im Falle von menschlichem oder tierischem Blut als Körperfluid sind Antikoagulantien umfassend EDTA, Citrat, Heparin und/oder deren Derivate. In einer besonders bevorzugten Ausführungsform ist die innere Oberfläche der ersten Kammer vergrößert, beispielweise mittels eines die innere Oberfläche der Kammer bearbeitenden Verfahrens, welches die Rauigkeit der inneren Oberfläche vergrößert. In einer besonders bevorzugten Ausführungsform ist in der ersten Kammer eine die innere Oberfläche verändernde und/oder vergrößernde Substanz angeordnet, die eine induzierende Wirkung auf die Bildung autologer Proteine haben kann. Die oberflächenverändernden und/oder oberflächenvergrößernden Substanzen umfassen Glasmehl, Glasgranulat, Quarzmehl, Quarzsand, Korund, Kügelchen, Perlen, Sand und Metalle. Die oberflächenverändernden und/oder oberflächenvergrößernden Substanzen umfassen auch organische Verbindungen und Polymere sowie biogene oder biologische Substanzen, wie beispielweise Zellulose, Collagene, Alginate, Nukleinsäuren und andere von Zellen gebildete Proteine oder Metabolite. Die oberflächenverändernde und/oder oberflächenmodifizierende Substanz kann von im Wesentlichen fester, flüssiger oder gelartiger Konsistenz sein. In einer weiteren besonders bevorzugten Ausführungsform sind Additive wie Antikoagulantien und/oder eine die innere Oberfläche verändernde und/oder vergrößernde Substanz in der ersten Kammer angeordnet. Alternativ sind keine Additive in der ersten Kammer vorgesehen.

Es ist möglich, dass in der ersten Kammer eine biologisch wirksame Substanz vorhanden ist oder diese in die erste Kammer mit dem schon eingefüllten Körperfluid eingebracht wird. Eine biologisch wirksame Substanz ist eine Substanz, die eine Veränderung der Physiologie bzw. des Metabolismus bewirkt. Beispiele für eine biologisch wirksame Substanz können Cortison, Gene oder DNA oder andere vorgenannte biogene oder biologische Substanzen sein.

In einer bevorzugten Ausführungsform ist mindestens ein Öffner vorgesehen, der derart ausgestaltet ist, zumindest einen zwischen der ersten und der zweiten Kammer angeordneten Teilfluidpfad auszubilden, indem der Öffner ein Element ist und/oder auf ein Element einwirkt das den Teilfluidpfad blockiert bzw. unterbricht. Bei dem Öffner kann es sich beispielweise um ein Durchstechmittel, zwischen der ersten und der zweiten Kammer handeln, das auf ein Septum einwirkt. Nicht limitierende Beispiele für einen Öffner umfassen ein Ventil, ein auf ein Ventil einwirkendes Element, einen Schieber, ein auf einen Schieber einwirkendes Element und/oder ein Durchstechmittel. Es kann vorgesehen sein, dass der Öffner Teil eines Verschlusses sein kann; beispielsweise kann der Öffner ein Betätigungsabschnitt eines Schiebers sein.

Der Öffner kann einen Verschluss der zweiten Kammer öffnen, insbesondere indem die zweite Kammer mittels eines Septums verschlossen ist, welches ein als Durchstechmittel zwischen der ersten Kammer und der zweiten Kammer ausgestalteter Öffner durchsticht. Das Durchstechmittel kann bevorzugt als ein- oder zweiendige Kanüle, ein- oder zweiendige Nadel und/oder ein oder mehrschneidige Klinge ausgestaltet sein. Bei Einbringen der erfindungsgemäßen Vorrichtung in eine handelsübliche Zentrifuge wie beispielsweise Festwinkel- oder Ausschwingzentrifugen kann in einer bevorzugten Ausführungsform der Öffner während einer Zentrifugation einen an der zweiten Kammer angebrachten Verschluss an einer Öffnung der zweiten Kammer öffnen. Vorzugsweise handelt es sich bei dem Öffner um ein Durchstechmittel und bei dem Verschluss der zweiten Kammer um eine auf die erste Kammer gerichtete endseitig angeordnete Dichtung, beispielweise einen Elastomerverschluss, der zweiten Kammer, mit der insbesondere eine Öffnung der zweiten Kammer wiederverschließbar ist.

Der Begriff "Elastomere" umfasst Grundstoffe, die sich bei Zug- oder Druckbelastung elastisch verformen, aber danach wieder in ihre im Wesentlichen ursprüngliche Gestalt zurückfinden.

Ferner kann, insbesondere zusätzlich zum Öffner und gegebenenfalls einem entsprechenden Element, auf das der Öffner einwirkt, zwischen der ersten und der zweiten Kammer die Barriere angeordnet sein. Die Barriere kann einen Fluss des Körperfluids aus der ersten Kammer in Richtung der zweiten Kammer verhindern. Die Barriere kann derart ausgestaltet sein, dass im Wesentlichen glatte Wandungen vorhanden sind, die mit dem Körperfluid in Kontakt kommen, so dass Öffnungen und/oder Unebenheiten, in die Bestandteile des Körperfluids in bestimmten Zuständen der Vorrichtung eindringen können, verringert und/oder vermieden werden. Die Barriere kann in Form eines Ventils ausgestaltet sein oder ein solches umfassen. Die Barriere kann einen Fluss des Körperfluids in Richtung der zweiten Kammer vor dem Öffner und dem Verschluss der zweiten Kammer verhindern. Die Barriere kann als rein physikalische Barriere bzw. Blockademittel, welche bzw. welches wahlweise schließt oder öffnet, ausgebildet sein. Die Barriere kann als Septum, welches mittels einer Nadel oder einem ähnlichen Durchstechmittel durchstochen werden kann, um einen Durchfluss zu ermöglichen, ausgestaltet sein. Ein Ventil als Barriere kann als Rückschlagventil oder "Duck bill valve" oder als Schirmventil, insbesondere angeordnet an der Außenseite einer für eine Befüllung vorgesehenen Öffnung gegenüberliegenden Wandung der ersten Kammer, ausgestaltet sein.

In einer bevorzugten Ausführungsform kann zwischen der ersten und zweiten Kammer ein Selektionsmittel angeordnet sein. Vorzugsweise kann das Selektionsmittel als ein Filter ausgestaltet sein. Ein geeigneter Filter kann ein Filter zur Größenselektion des durchdringenden Fluids sein. Es kann eine Porengröße eines Filters von im Wesentlichen 100 µm, 90 µm, 20 µm, 5 µm, 3,2 µm, 1 µm, 0,4 µm, 0,2 µm oder 0,1 µm gewählt werden. Alternativ sind Ionenaustauscher oder Sorbentien mit angebrachten Liganden möglich.

Vorzugsweise sind die Barriere und der Öffner derart angeordnet, dass der Öffner näher zur zweiten Kammer angeordnet ist als die Barriere. Die Barriere kann den Öffner vor dem Eintritt des Körperfluids schützen. Die Barriere kann auch den Austritt aus der ersten Kammer verhindern. Der Filter kann zwischen der Barriere und dem Öffner angeordnet sein. Der Öffner kann einen Verschluss der zweiten Kammer öffnen. Die Barriere, insbesondere in Form eines vorgenannten Rückschlagventils oder Schirmventils, kann insbesondere bei einem längsaxialen Verschieben geöffnet werden und der Öffner bei diesem längsaxialen Verschieben die zweite Kammer öffnen. Hierdurch kann eine Verbindung zwischen der ersten und der zweiten Kammer hergestellt werden, bei der zunächst mindestens zwei oder genau zwei Verschlüsse, zum Einen die Barriere und zum Anderen der Verschluss der zweiten Kammer, verwendet werden, um eine Sterilität weitestgehend sicherzustellen.

In einer bevorzugten Ausführungsform weist die zweite Kammer einen Unterdruck auf, wodurch der Transfer zumindest eines Teils des Körperfluids zwischen der ersten und der zweiten Kammer vereinfacht werden kann. Es ist eine einfache Ausgestaltung einer zweiten Kammer mit konstanter effektiver Größe möglich. Ein Bewegen von Wandungen der Kammer zueinander, welches eine Veränderung der effektiven Größe der Kammer bewirkt, kann entfallen. Es kann alternativ oder zusätzlich vorgesehen sein, dass ein Luftauslass an der zweiten Kammer vorgesehen ist, der für einen Druckausgleich der zweiten Kammer ausgestaltet sein kann. Besonders bevorzugt kann der Luftauslass in Art einer Rückschlagarmatur ausgestaltet sein, bei der ein Schließelement in einer Richtung zwangsgeschlossen ist und in der anderen Richtung einen Fluss der Luft freigibt. Beispielsweise kann eine derartige Rückschlagarmatur eine Feder aufweisen, die das Schließelement in einer Richtung durch die Feder schließt und in der anderen Richtung den Luftdurchfluss von der Kammer nach außen freigibt. Als Schließelement kommen Kegel, Kugel, Klappe und/oder Membran in Betracht, die jeweils in einen entsprechenden Sitz gedrückt werden. Steht in der Richtung, in der das Schließelement einen Fluss freigibt ein Druck an, der die Kraft der Feder übersteigt, wird das Schließelement vom Sitz abgehoben und der Durchfluss ist frei. Es sind auch Ausführungsformen möglich, in denen keine Feder verwendet wird, beispielsweise eine Ausführungsform, in der das Schließelement im Wesentlichen nur durch einen höheren anstehenden Außendruck eines Fluids, die strömende Luft oder die Gewichtskraft des Schließelements schließt.

Die zweite Kammer ist verschieblich gegenüber der Längsachse der ersten Kammer verschiebbar. Die zweite Kammer ist in einer Führung verschieblich angeordnet. Es ist ein Blockiermittel vorgesehen, dass die Relativbewegung von zweiter Kammer und erster Kammer zueinander verhindert. Das Blockiermittel kann als ein Anschlagelement ausgestaltet sein, das zur Aufhebung der Blockierung entfernt oder in eine andere Position gegenüber einem Stützelement gebracht werden kann. In einer bevorzugten Ausführungsform kann die Blockierung selbsttätig während einer Zentrifugation der Vorrichtung aufgehoben werden. In einer alternativen, besonders bevorzugten Ausführungsform kann die Blockierung durch den Anwender vor der Zentrifugation aufgehoben werden. Geeignete Blockiermittel im Sinne der Beschreibung umfassen Abbrechlaschen oder kompressierbare Federn. Die zweite Kammer kann relativ zur ersten Kammer verschieblich bewegt werden, wobei die zweite Kammer sich auf die erste Kammer zu bewegen kann. Neben der relativen Bewegung der ersten Kammer und zweiten Kammer kann die zweite Kammer bevorzugt eine Relativbewegung in bzw. an der Führung ausführen. Die Relativbewegung zwischen erster und zweiter Kammer kann durch eine Bewegung der Führung, die Teil eines Kolbens sein kann, bewirkt werden, indem die zweite Kammer zusammen mit der Führung relativ zur ersten Kammer bewegt wird. Die Vorrichtung kann mehrere Relativbewegungen ermöglichen: a) Zweite Kammer und Führung bewegen sich zusammen relativ zu ersten Kammer und b) zweite Kammer bewegt sich relativ zur Führung. Die zweite Kammer kann in einer besonders bevorzugten Ausführungsform als Behältnis, welches insbesondere einen Unterdruck aufweisen kann (beispielsweise als vacuum vial), ausgestaltet sein, welches in der Führung gelagert ist. Das Behältnis (zweite Kammer) kann von der Führung getrennt werden, nachdem eine Überführung von Körperfluid in die zweite Kammer erfolgte. Hierzu kann das Behältnis - eventuell zusammen mit einer Lagerung - aus der Führung entlang der Längsachse der Vorrichtung gezogen werden. Eine eventuell vorhandene Lagerung kann vom Behältnis (zweite Kammer) entfernt werden. Zur Verbesserung der Handhabung kann die Lagerung einen Stempel oder Griff aufweisen, der sich endseitig an der Lagerung entlang der Längsachse der Vorrichtung erstreckt.

Es kann vorgesehen sein, dass die zweite Kammer eine Einfüllöffnung und eine zur Einfüllöffnung separate Entnahmeöffnung aufweist, die insbesondere je ein Septum aufweisen können. Hierdurch kann sichergestellt werden, dass die Einfüllöffnung nicht auch als Entnahmeöffnung verwendet werden muss, was eine Kontaminationsgefahr weiter verringert.

In einer Ausführungsform kann der Öffner und/oder eine Öffnung an der zweiten Kammer im Wesentlichen mittig bezogen auf den Querschnitt der Vorrichtung ausgebildet bzw. angeordnet sein. Hierdurch kann ein einfacher Aufbau ermöglicht werden. Es kann jedoch auch vorgesehen sein, dass der Öffner und/oder eine Öffnung an der zweiten Kammer außenmittig bezogen auf den Querschnitt der Vorrichtung ausgebildet bzw. angeordnet sind, was bei Festwinkelrotoren vorteilhaft sein kann.

Der Begriff "Feder" im Sinne der Beschreibung umfasst ein technisches Bauteil, das sich elastisch verformen lässt. Nicht limitierende Beispiele für Federn sind Schraubenfedern, Zugfedern und Stabfedern.

Die erste Kammer weist einen Zylinder als zylindrischen Abschnitt und eine in dem Zylinder beweglich verschiebbare Führung, die die verschiebliche Wandung umfasst, auf. Die zweite Kammer kann als Behältnis an der Führung derart gelagert sein, dass das Behältnis bei der Zentrifugation relativ zur Führung in Richtung der ersten Kammer bewegt wird, um Körperfluid von der ersten Kammer in das Behältnis zu überführen. Die zweite Kammer kann sich in Längsrichtung der ersten Kammer bewegen. Die zweite Kammer kann eine konstante effektive Größe aufweisen.

Der Begriff "Führung" im Sinne der Beschreibung umfasst eine mit der verschieblichen Wandung verbundene Führungsfläche. Die Führungsfläche erstreckt sich im Wesentlichen parallel zur Längsachse der Vorrichtung. Entlang der Führungsfläche kann die zweite Kammer in Längsrichtung der Vorrichtung bewegt werden. Die Führungsfläche ist als zumindest abschnittsweise ausgebildete Mantelfläche eines Zylinders, die konzentrisch zur Mantelfläche des zylindrischen Abschnitts der ersten Kammer ausgerichtet ist, ausgebildet. Die Führung kann die zweite Kammer gleitverschieblich in Richtung der Längsachse der Vorrichtung aufnehmen; die Führungsfläche kann eine innere Mantelfläche sein. Die zweite Kammer kann unmittelbar oder mittelbar an der Führung gleiten. Im mittelbaren Falle kann die zweite Kammer in einer Halterung gehalten werden, die an der Führungsfläche der Führung gleiten kann. Zwischen der Halterung oder der zweiten Kammer und der Führungsfläche der Führung ist mindestens ein Dichtmittel in Umfangsrichtung angeordnet.

Der Begriff "Halterung" im Sinne der Beschreibung umfasst ein technisches Bauteil, das zur Aufnahme der zweiten Kammer geeignet ist. Im Sinne der Beschreibung kann die Halterung als ein klammerartiges Bauteil ausgestaltet sein, welches eine als Behältnis ausgestaltete zweite Kammer umgreift. Die Halterung kann eine Öffnung aufweisen, die in Richtung einer Öffnung der zweiten Kammer gerichtet ist. Die Halterung kann eine zumindest abschnittsweise ausgestaltete im Wesentlichen zylinderförmige Mantelfläche aufweisen, die die zweite Kammer zumindest abschnittsweise umfangsseitig umgibt. Ferner kann die Halterung eine Basis aufweisen, die zur Anlage einer Außenseite eines Bodens der zweiten Kammer eingerichtet ist. Die Innenseite der Mantelfläche der Halterung kann bezogen auf ihren Innendurchmesser auf einen Außendurchmesser der zweiten Kammer angepasst sein. An dem der Basis gegenüberliegenden Ende können auf einer Höhe der zweiten Kammer angepasste Rastvorsprünge vorgesehen sein, so dass die zweite Kammer in Form einer Art eines Formschlusses in der Halterung gehalten wird. Die Halterung kann mit ihrer zumindest abschnittsweise als Mantelfläche eines Zylinders ausgestalteten Seitenwand an der Führung gleiten. Zur Verringerung des Kontakts zwischen der Führung und der Halterung können an der Halterung Vorsprünge ausgebildet sein, die an der Führungsfläche der Führung gleiten können. Insbesondere kann zwischen den Vorsprüngen eine Dichtung zwischen der Führung bzw. Führungsfläche und der Außenseite der Halterung ausgebildet sein.

In einer besonders bevorzugten Ausführungsform der Erfindung kann sich die Führung zusammen mit der verschieblichen Wandung der ersten Kammer relativ zur ersten Wandung - unter Veränderung der Größe der ersten Kammer - bewegen. Ferner kann sich die Halterung der zweiten Kammer relativ zu der Führung und damit auch relativ zu der ersten Wandung - ohne Veränderung der Größe der ersten Kammer - bewegen.

Die erste Kammer weist einen als Zylinder ausgebildeten zylindrischen Abschnitt auf und eine im Zylinder verschieblich gelagerte Wandung auf. Ferner kann die erste Kammer eine Öffnung an einem Verbindungsteil, insbesondere mit einem Septum, aufweisen. Das Verbindungsteil mit der Öffnung ist insbesondere außen an einer Querfläche des Zylinders, die der verschieblichen Wandung gegenüberliegt, ausgebildet. Die verschiebliche Wandung kann ein Ventil aufweisen, welches insbesondere bei einem Befüllen der ersten Kammer durch Verschiebung der verschieblichen Wandung zur Vergrößerung der ersten Kammer geschlossen ist und zur Überführung des Körperfluids in die zweite Kammer geöffnet wird. Die verschiebliche Wandung ist mit der Führung verbunden. Die Führung und die verschiebliche Wandung können derart ausgestaltet in dem Zylinder angeordnet sein, dass sie zusammen in dem Zylinder verschoben werden können, wobei die Führung an der der Öffnung der ersten Kammer gegenüberliegenden Seite zur verschieblichen Wandung angeordnet ist. Die verschiebliche Wandung weist zur Innenfläche des Zylinders, in dem sie verschieblich gelagert ist, mindestens ein Abdichtelement in Form eines O-Rings auf. Die verschiebliche Wandung kann insbesondere eine Öffnung mit einem Ventil aufweisen. Die verschiebliche Wandung kann ferner ein Selektionsmittel, insbesondere in Form eines Filters, welches zwischen der ersten Kammer und der zweiten Kammer angeordnet ist, aufweisen. Der Filter kann fest mit der verschieblichen Wandung verbunden sein. Zwischen dem Filter und der zweiten Wandung kann ein Öffner in Form eines Durchstechmittels, insbesondere in Form einer Kanüle, deren Spitze in Richtung der zweiten Kammer ausgerichtet ist, angeordnet sein. Die verschiebliche Wandung, der Filter, der Öffner und die Führung können als Teil eines Kolbens, der in dem Zylinder bzw. zylindrischen Abschnitt verschieblich bewegt werden kann, ausgestaltet sein oder einen Teil eines verschieblichen Zylinders, der in dem zylindrischen Abschnitt bzw. dem Zylinder verschieblich gelagert ist, bilden. Verschiebliche Wandung, Filter, Öffner und Führung können als Ganzes gehandhabt werden und in dem Zylinder bzw. zylindrischen Abschnitt als Ganzes bewegt werden. Insbesondere kann ein, insbesondere als Druckelement an der Führung ausgestaltetes, Betätigungselement vorgesehen sein, welches mit einer, insbesondere als Ventil ausgestalteten, Barriere, an der verschieblichen Wandung derart zusammenwirken kann, dass bei Ausübung eines Drucks, beispielsweise mittels der zweiten Kammer das Ventil öffnet. Bei Aufheben der Wirkung der Barriere, d.h. insbesondere Ausüben eines Drucks auf ein Verschlussteil des Ventils, kann die Barriere, insbesondere das Ventil, öffnen und die beiden Kammern miteinander in Fluidverbindung bringen. Die Barriere bzw. das Ventil kann auch ohne ein Betätigungselement ausgestaltet sein. Das Ventil kann beispielsweise aufgrund eines am Ventil anstehenden "Überdrucks" öffnen. Das Öffnen der Barriere geschieht vorzugsweise dann, wenn die zweite Kammer, die im bzw. an der Führung gelagert ist, sich zur Führung derart bewegt hat, dass das Durchstechmittel ein an einer Öffnung der zweiten Kammer vorgesehenes Dichtmittel durchstochen hat. Die zweite Kammer ist vorzugsweise als Behältnis, in dem ein Unterdruck vorliegt, ausgestaltet. Die zweite Kammer kann derart in bzw. an der Führung gelagert sein, dass sich die zweite Kammer auf das Durchstechmittel zubewegen kann, um eine Fluidverbindung zwischen der ersten Kammer und der zweiten Kammer herzustellen. Hierzu kann eine Ausführungsform vorgesehen sein, bei der die zweite Kammer auf das Durchstechmittel zubewegt wird, um das Dichtmittel der zweiten Kammer zu durchstechen und die zweite Kammer mittelbar oder unmittelbar in einer weiteren oder in der gleichen Bewegung auf ein Druckmittel drückt, welches das Ventil, welches an der verschieblichen Wandung ausgebildet ist, öffnet. Das Ventil kann auch derart ausgestaltet sein, das dieses bei einem Überdruck in der ersten Kammer öffnet. Das Ventil kann zusätzlich als Rückschlagventil ausgestaltet sein. Vorzugsweise kann die zweite Kammer zusammen mit der Führung und damit zusammen mit der verschieblichen Wandung, dem Filter und dem Durchstechmittel bewegt werden. Die zweite Kammer kann aber auch zusätzlich in der Führung gelagert sein, um eine Relativbewegung zur Führung durchzuführen. Hierzu kann die zweite Kammer als Teil eines Kolbens, der sich in einem zylindrischen Abschnitt einer Führung bewegen kann, ausgestaltet sein. Die Führung, die Teil eines Kolbens im zylindrischen Abschnitt der ersten Kammer ist, kann insbesondere ihrerseits einen zylindrischen Abschnitt aufweisen, in dem die zweite Kammer als Kolben ausgebildet ist. Insbesondere kann die als Behältnis ausgestaltete zweite Kammer gegenüber einer Innenwandung eines zylindrischen Abschnitts der Führung mittels eines oder mehrerer Dichtmittel, die insbesondere als O-Ring ausgestaltet sein können, abgedichtet sein. Es ist vorgesehen, dass eine Bewegung der zweiten Kammer innerhalb der Führung mittels eines Blockiermittels verhindert werden kann. Nach Entfernen des Blockiermittels und/oder außer Eingriff bringen des Blockiermittels kann eine Bewegung der zweiten Kammer relativ zur Führung erfolgen. Die Bewegung der Führung relativ zum zylindrischen Abschnitt bzw. Zylinder der ersten Kammer und die Bewegung der zweiten Kammer relativ zur Führung können im Wesentlichen auf der gleichen Längsachse erfolgen. Vorzugsweise kann nach Aufheben der Blockade der Bewegung des als Behältnis ausgebildeten zweiten Kammer, eine verschiebliche Bewegung der zweiten Kammer relativ zur Führung erfolgen, bei der in einem ersten Schritt zur Verbindung der ersten und der zweiten Kammer das Dichtmittel der zweiten Kammer vom Durchstechmittel durchstochen werden kann, indem die zweite Kammer relativ zur Führung bewegt wird und in einem nächsten Schritt, bei dem die zweite Kammer zusammen mit der Führung bewegt wird, das Ventil geöffnet werden. Aufgrund der bei der Zentrifugation wirkenden Kräfte wird bei der Zentrifugation neben einer Separation von Phasen des in der ersten Kammer enthaltenen Körperfluids eine Bewegung der zweiten Kammer in der Führung initiiert, die im Wesentlichen quer zu den ausgebildeten Phasengrenzen und/oder in Richtung der Längsachse der Vorrichtung ist. Durch die Bewegung der zweiten Kammer wird diese zunächst durch das Durchstechmittel geöffnet und dann das Ventil zwischen der ersten und der zweiten Kammer geöffnet. Ferner bewegt sich die Führung zusammen mit der verschieblichen Wandung in der gleichen Richtung wie die zweite Kammer zusammen mit dieser, so dass ein Druck auf das in der ersten Kammer befindliche Körperfluid ausgeübt wird und das Körperfluid aus der Kammer herausgedrückt und in die zweite Kammer mittels des Unterdrucks gesaugt wird. Die Bewegung und die Lagerung der Führung im zylindrischen Abschnitt der ersten Kammer und die Lagerung und Anordnung der zweiten Kammer in der Führung sind derart eingerichtet bzw. aufeinander abgestimmt, dass die beschriebenen relativen Bewegungen und insbesondere eine Überführung einer sich während der Zentrifugation ergebenden Phase in die zweite Kammer erfolgen. Die Vorrichtung kann nach der Zentrifugation zur Separierung der Phasen aus der Zentrifuge entnommen und ferner die zweite Kammer aus der Führung entfernt werden, wobei eine Bewegung ausgeführt wird, die entgegengesetzt der Bewegung ist, die zum Durchstechen des Dichtmittels geführt hat und dabei wird das Dichtmittel wieder geschlossen. Die zweite Kammer kann in einer gewünschten Art und Weise gehandhabt werden.

Es wird ein Verfahren beschrieben, bei dem Körperfluid von einer ersten Kammer in eine zweite Kammer überführt wird, wobei die effektive Größe der ersten Kammer mittels einer verschieblichen Wandung verändert wird und die zweite Kammer relativ zur verschieblichen Wandung bewegt wird. Insbesondere können die erste und die zweite Kammer bei einer Zentrifugation verbunden werden und das Körperfluid bei Zentrifugation mittels der dabei wirkenden Kräfte aus der ersten Kammer in die zweite Kammer überführt werden.

Es kann vorgesehen sein, dass ein Überführen zumindest eines Teils des Körperfluids durch ein Verschieben der zweiten Kammer in Richtung der ersten Kammer erfolgt. Während des Verschiebens kann ein Druckausgleich ausgeführt werden. Alternativ oder zusätzlich ist es möglich, dass das Verschieben druckausgleichsfrei erfolgt. Im Falle eines druckausgleichsfreien Verschiebens müssen keine Mittel vorgesehen werden, die für einen Druckausgleich verwendet werden müssen; die Vorrichtung kann vom konstruktiven Aufbau und/oder der Anzahl der Bauelemente vereinfacht werden. Im Falle eines Verschiebens mit einem Druckausgleich kann der Vorgang des Verschiebens möglicherweise einfacher ausgeführt werden, da kein erhöhter Druck in Schieberichtung vorliegt.

Der Begriff "Zentrifugation" im Sinne der Beschreibung umfasst den Vorgang des Zentrifugierens einer Flüssigkeit in einer Zentrifuge, wie sie gemeinhin als Festwinkel- oder Ausschwingzentrifuge bezeichnet wird. Beispielsweise kann die Vorrichtung in Zentrifugen für 50 ml Falcon tubes eingebracht werden. Die Dauer der Zentrifugation kann zwischen einer bis 90 Minuten und bevorzugt zwischen einer bis 60 Minuten variiert werden. Es kann vorgesehen sein, dass das Körperfluid vor der Zentrifugation oder während der Zentrifugation inkubiert wird, wobei bevorzugt bei einer Inkubation während der Zentrifugation - eventuell bei niedrigeren Umdrehungszahlen - eine längere Dauer der Zentrifugation möglich ist. Geeignete relative Zentrifugalbeschleunigungen (RZB), mit denen das Verfahren durchgeführt werden kann, liegen im Bereich von 10 bis 10.000 G, bevorzugt 10 bis 5.000 G, besonders bevorzugt 10 bis 4.000 G. Nach der Zentrifugation kann die in der zweiten Kammer befindliche Phase des ursprünglich eingebrachten Körperfluids sofort verwendet werden. Alternativ kann die Körperfluidphase in der zweiten Kammer gekühlt, eingefroren und/oder lyophilisiert oder anderweitig verarbeitet werden. Im Sinne der Beschreibung umfasst eine "anderweitige Verarbeitung" das Hinzufügen von Additiven, des Portionieren in Kapseln, das Beschichten von zu implantierenden Vorrichtungen mit dem Körperfluid der zweiten Kammer und weitere mögliche Verwendungen. Nachfolgend wird eine Ausführungsform eines Verfahrensablaufs beschrieben:
Die erste Kammer kann mittels Bewegung einer verschieblichen Wandung der ersten Kammer mit Blut befüllt werden. Vorzugsweise wird einem Organismus das Blut direkt mit der die erste Kammer umfassenden Vorrichtung entnommen. In einer besonders bevorzugten Ausführungsform wird die erste Kammer mit 1 bis 25 ml, bevorzugt 5 bis 20 ml und besonders bevorzugt 10 bis 15 ml Blut befüllt. Anschließend kann eine erste Inkubation der mit Blut befüllten Vorrichtung durchgeführt werden, an die sich eine Zentrifugation anschließt. Alternativ kann die mit Blut gefüllte Vorrichtung auch direkt nach Befüllung zentrifugiert werden, wobei eine Inkubation während des Zentrifugierens durchgeführt werden kann.

Der Begriff "Inkubation" im Sinne der Beschreibung umfasst eine definierte Zwischenlagerung oder Lagerung der Vorrichtung unter definierten Bedingungen, die im Wesentlichen die Temperatur und die Zeit sind. Eine Zwischenlagerung oder Lagerung kann auch innerhalb der Zentrifuge erfolgen.

Die Dauer einer Inkubation kann variiert werden, ebenso wie die Temperatur. Vorzugsweise sind die innere Oberfläche verändernde und/oder vergrößernde Strukturen in der ersten Kammer vorgesehen, die eine induzierende Wirkung auf die Bildung autologer Proteine haben können. Ein nicht limitierendes Beispiel für die Anreicherung autologer Proteine ist der Interleukin 1-Rezeptorantagonist (IL-1Ra). Für die Inkubation zur Anreicherung von IL-1RA geeignete Bedingungen sind dem Fachmann unter anderem aus EP 1 151 004 B1 bekannt. Exemplarisch seien genannt eine Inkubation für 24 Stunden bei 37 bis 41 °C und eine Inkubation für 12 bis 72 Stunden bei Raumtemperatur. Nach einer Inkubation kann sich in dem Beispiel eine Zentrifugation zur Separation einzelner Blutbestandteile anschließen. Das Verfahren betrifft hierbei eine Zentrifugation, bei der eine Blutphase während der Zentrifugation in eine erste Kammer transferiert wird. Vorzugsweise handelt es sich bei der transferierten Blutphase um Blutplasma oder -serum. In einer besonders bevorzugten Ausführungsform ist die zu transferierende Blutphase angereichert mit autologem IL-1Ra.

Es wird auch eine Verwendung von bei einer Zentrifugation wirkenden Kräften beschrieben, die zur Separation von Phasen eines Körperfluids verwendet werden, wobei die Kräfte ferner dazu verwendet werden, um eine erste Kammer und eine zweite Kammer zu verbinden und das Körperfluid aus der ersten Kammer in die zweite Kammer zu überführen.

Zahlenangaben umfassen neben dem angegebenen Zahlenwert auch insbesondere toleranzbedingte Abweichungen von +/- 20 %, besonders bevorzugt +/- 10 % und damit einen entsprechenden Wertebereich.

Die Ausführungen in der Beschreibung hinsichtlich der Aspekte der Vorrichtung, des Verfahrens und der Verwendung ergänzen einander, so dass Ausführungen zu einem Aspekt auch für den anderen Aspekt gelten.

Die Erfindung wird nachfolgend anhand eines in den Zeichnungen dargestellten Ausführungsbeispiels näher erläutert.

Die vorstehenden Ausführungen stellen ebenso wie die nachfolgende Beschreibung beispielhafter Ausführungsformen keinen Verzicht auf bestimmte Ausführungsformen oder Merkmale dar.

In den Zeichnungen zeigt:
- Fig. 1:: Eine Vorrichtung in einer isometrischen Darstellung schräg von oben;
- Fig. 2:: die Vorrichtung gemäß Fig. 1 in einer Grundstellung in teilweise geschnittener Darstellung;
- Fig.3:: die Vorrichtung gemäß Figur 2 nach Befüllen;
- Fig.4:: die Vorrichtung gemäß Figur 3 nach einer Zentrifugation; und
- Fig.5:: eine Detailansicht der Vorrichtung.

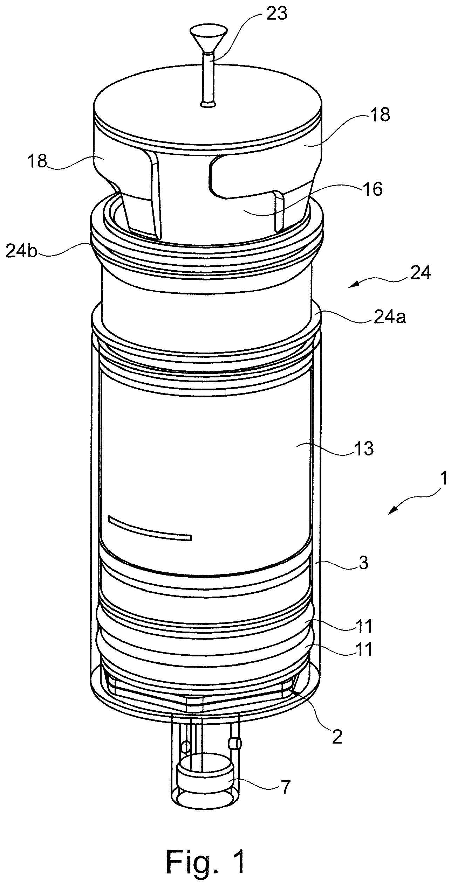

Fig. 1 zeigt in einer isometrischen Darstellung eine Vorrichtung 1, die in Bezug auf die nachfolgenden Figuren näher beschrieben wird.

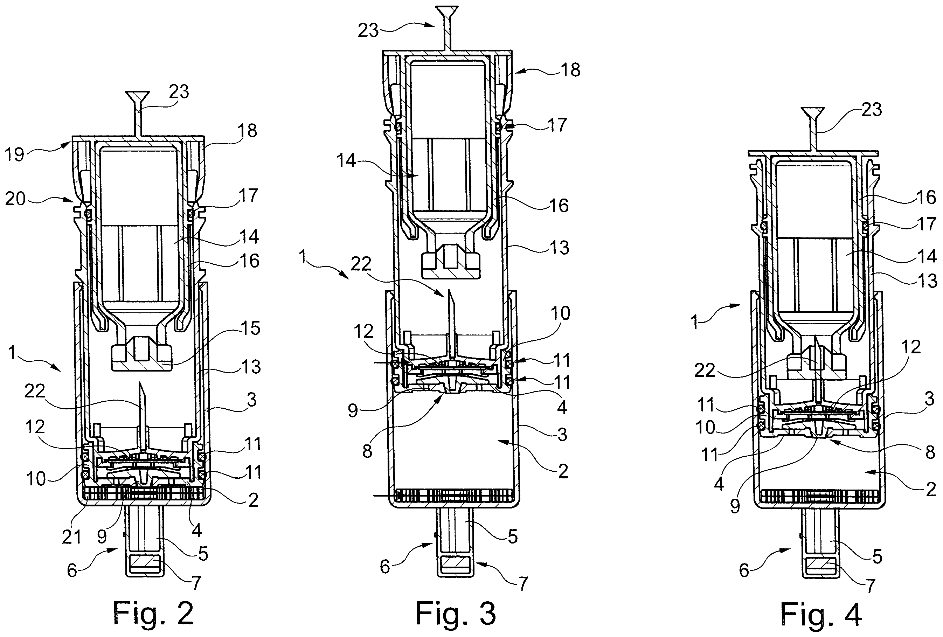

Figur 2 zeigt in einer schematischen Darstellung die Vorrichtung 1 in teilweise geschnittener Darstellung. Die Vorrichtung 1 ist in einem ersten Zustand, der einer Grundstellung bei der Auslieferung der Vorrichtung 1 und der Darstellung in Fig. 1 entspricht.

Die Vorrichtung 1 weist eine erste Kammer 2 auf, die ein veränderliches effektives Volumen hat. Die erste Kammer 2 weist einen zylindrischen Abschnitt bzw. Zylinder 3 auf, in dem eine Wandung 4 verschieblich gelagert ist. In dem in der Figur 1 dargestellten Ausführungsbeispiel ist der Zylinder 3 ein Kreiszylinder und die Wandung 4 eine an die Innenabmessung des Zylinders 3 angepasste Kreisfläche. Die Wandung 4 ist entlang einer Längsachse des Zylinders 3 verschieblich. Die Kammer 2 weist eine Öffnung 5 auf, die an einer Querfläche des Zylinders 3 ausgebildet ist. Im Bereich der Öffnung 5 ist ein Verbindungsteil 6 vorgesehen, das für ein Verbinden mit einem externen Element, wie einer Kanüle oder Nadel, ausgestaltet ist. Im Bereich der Öffnung 5 ist ein Septum 7 vorgesehen, mit dem eine Verbindung mit der ersten Kammer 2 reversibel hergestellt und wieder geschlossen werden kann. Mittels des externen Elements und der Öffnung 5 kann die erste Kammer 2 mit Körperfluid gefüllt werden.

Die Wandung 4 weist mindestens ein eine Öffnung 8, im dargestellten Falle mehrere Öffnungen 8 auf, welche mittels einer als (Schirm-)Ventil ausgestalteten Barriere 9 wahlweise geschlossen oder geöffnet werden können. Die Wandung 4 weist ferner einen sich in der Richtung der Verschiebung (Längsachse) erstreckenden Abschnitt 10 auf, an dem Dichtungen 11 vorgesehen sind, die die erste Kammer 2 bei der Bewegung in Bezug auf die Wandung 4 abdichten. Die Dichtungen 11 sind in Form von O-Ringen, die sich in Umfangsrichtung um die Längsachse erstrecken, ausgebildet. Die Dichtungen 11 sind in Aufnahmen des Abschnitts 10 angeordnet. Die Dichtungen 11 stellen eine Dichtung zwischen der Wandung und dem Zylinder 3 her.

Mit der Wandung 4 ist ein als Filter ausgestaltetes Selektionsmittel 12 verbunden, das zusammen mit der als Ventil ausgestalteten Barriere 9 in Längsrichtung des Zylinders 3 verschoben werden kann. Die Wandung 4 ist mit einer Führung 13 verbunden, die ihrerseits in dem im Ausführungsbeispiel gezeigten Falle als zylinderförmiges Element ausgestaltet ist und sich von der Wandung 4 weg erstreckt.

An der Führung 13 ist eine zweite Kammer 14 angeordnet, die in der Führung 13 in Längsachse des Zylinders 3 und der Führung 13 verschoben werden kann. Die zweite Kammer 14 ist als ein Behältnis ausgeführt, welches einen Unterdruck aufweist. Die zweite Kammer weist eine konstante effektive Größe auf. Die zweite Kammer 14 ist mittels eines Dichtmittels 15 verschlossen. Die zweite Kammer 14 wird in der Führung 13 von einer Halterung 16 gehalten, die eine gegenüber den Innenabmessungen der Führung 13 angepasste Außenabmessung aufweist. Die Halterung 16 ist gleitverschieblich in der Führung 13. Zum Abdichten nach außen weist die Halterung 16 einen in einer umfänglichen Nut um die Halterung 16 angeordneten O-Ring 17 auf.

Im in den Figuren 1 bis 3 dargestellten Zustand ist zur Verhinderung einer relativen Bewegung entlang der Längsachse der Vorrichtung 1 zwischen der Halterung 16 und damit der zweiten Kammer 14 gegenüber der Führung 13 ein Blockiermittel 18 zwischen Führung 13 und Halterung 16 vorgesehen, welches als Abbrechlasche ausgeführt ist. Das Blockiermittel 18 ist zwischen einem sich in Querrichtung der Längsachse erstreckenden Anschlagabschnitt 19 der Halterung 16 und einem endseitigen Abschnitt der Führung 13 angeordnet.

In Figur 3 ist der Zustand der Vorrichtung 1 gemäß Figur 1 bzw. 2 in einem Zustand nach einem Befüllen mit Körperfluid, hier Blut gezeigt. Die Befüllung der Vorrichtung 1, hier der ersten Kammer 2 erfolgt durch ein Verbinden des Verbindungsteils 6 mit einer Kanüle und einem Bewegen der Wandung 4 zur Vergrößerung der effektiven Größe der ersten Kammer 2, wobei die Kanüle in ein mit Blut gefülltes Lumen eines menschlichen oder tierischen Körpers endseitig gestochen wurde. Das Körperfluid wird in die erste Kammer 2 gesaugt. Die Wandung 4 wird dadurch bewegt, dass ein Griffabschnitt 24, der quer zur Längsachse der Vorrichtung 1 an der Führung 13 ausgebildet ist, ergriffen wird und die Wandung 4 entlang der Längsachse nach hinten gezogen wird. Der Griffabschnitt 24 ist durch eine Längsrichtung beabstandete Vorsprünge 24a, 24b begrenzt. Die Vorsprünge 24a, 24b verlaufen entlang des Umfangs um die Führung 13. Beim Befüllen mit Körperfluid ist die als Schirmventil ausgestaltete Barriere 9 geschlossen, so dass die erste Kammer 2 das Körperfluid aufnimmt und, wenn die Kanüle wieder vom Verbindungsteil 6 getrennt wird, das Körperfluid steril in der ersten Kammer 2 ist. Für eine sterile Inkubation ist in der ersten Kammer 2 eine die innere Oberfläche vergrößernde bzw. verändernde Substanz 21 vorgesehen, die eine indizierende Wirkung auf die Bildung autologer Proteine umfassen kann.

Nach oder während der sterilen Inkubation kann eine Zentrifugation erfolgen. Der sich dabei ergebende Zustand der Vorrichtung 1 ist in Figur 4 dargestellt. Vor der Zentrifugation wurde das Blockiermittel 18 entfernt.

Die Figur 4 zeigt den Zustand der Vorrichtung 1 nach der Zentrifugation, bei der zum einen das Körperfluid, hier Blut, in die einzelnen Phasen getrennt wurde und durch die bei der Zentrifugation auftretenden Kräfte eine Bewegung der Führung 13, der Wandung 4 und der zweiten Kammer 14 induziert wurde. Hierdurch kann das Körperfluid in einzelne Phasen getrennt werden und insbesondere eine der Phasen in die zweite Kammer 14 während der Zentrifugation überführt werden. Durch das Entfernen des Blockiermittels 18 kann eine Bewegung der zweiten Kammer 14 bzw. der Halterung 16 für die zweite Kammer 14 relativ zur Führung 13 erfolgen, so dass die Halterung 16 zusammen mit der zweiten Kammer 14 sich entlang der Längsachse der Führung 13 bzw. auf die erste Kammer 2 zubewegt, wobei das Dichtmittel 15 der zweiten Kammer 14 von einem Durchstechmittel ausgestalteten Öffner 22 durchstochen wird und somit die zweite Kammer 14 geöffnet wird. Die zweite Kammer 14 ist damit fluidisch mit dem Filter 12 und dem Ventil 9 verbunden.

Durch das Absinken der Halterung 16 und der zweiten Kammer 14 kann ein Druck auf die Barriere 9 ausgeübt werden, so dass die als Ventil ausgestaltete Barriere 9 die Öffnungen 8 der ersten Kammer 2 öffnet und damit die erste Kammer 2 und die zweite Kammer 14 miteinander verbunden sind. Durch die bei der Zentrifugation wirkenden Kräfte wird die Führung 13 zusammen mit der zweiten Kammer 14 und der Wandung 4 in Richtung auf die Öffnung 5 verschoben, so dass die obere der von dem Körperfluid abgetrennten Phasen bei der Bewegung, die durch die Zentrifugationskräfte bewirkt wird, überführt wird. Dieser Zustand ist für das Ende der Überführung in der Figur 4 dargestellt.

Nach dem Zentrifugieren kann das Behältnis in Form der zweiten Kammer 14 aus der Führung 13 entfernt werden. Hierzu kann ein Anwender die Vorrichtung 1 an einem Griff 23, der endseitig an der Halterung 16 ausgebildet ist, ergreifen und aus der Zentrifuge nehmen. Die Vorrichtung 1 kann dann an dem Griffabschnitt 24 seitlich gehalten werden und die Halterung 16 aus der Führung 13 gezogen werden. Bei der Entfernung durch eine längsaxiale Bewegung der Halterung 16 mit der Kammer 14 von dem Öffner 22 weg, wird das Dichtmittel 15 der zweiten Kammer 14 wieder geschlossen. Das Behältnis in Form der zweiten Kammer 14 kann aus der Halterung 16 entnommen werden. Die in der zweiten Kammer 14 befindliche abgetrennte Phase wurde steril prozessiert und steril von der ersten Kammer 2 in die zweite Kammer 14 überführt, ohne dass es eines Verfahrensschrittes durch einen Anwender bedurfte.

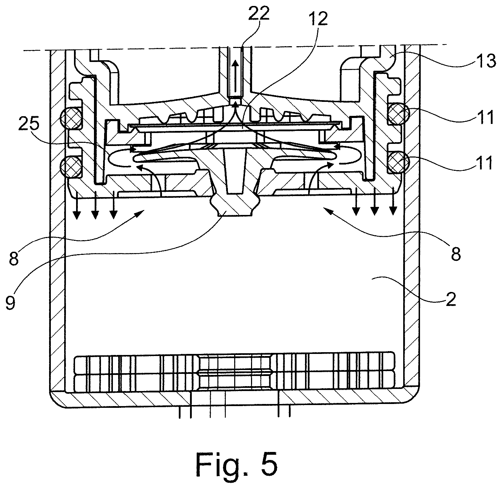

Fig. 5 zeigt eine Detailansicht der Vorrichtung 1. Mit den geschwungenen Pfeilen 25 ist schematisch ein Fluidpfad angegeben, der sich ergibt, wenn die Barriere 9 geöffnet ist. Die als Schirmventil ausgestaltete Barriere 9 gibt die Öffnungen 8 in der Wandung 4 frei. Die Freigabe der Öffnungen 8 beruht im Wesentlichen aufgrund des Drucks des Körperfluids, welcher sich durch die Verschiebung der Wandung 4 in Richtung auf die Öffnung 5 ergibt; die Verschiebung der Wandung 4 ist mittels der drei Pfeile rechts und links angegeben. Fluid fließt von der ersten Kammer 2 durch die Öffnungen 8, durch das Selektionsmittel 12, und weiter durch den Öffner 22 in die zweite Kammer 14.

## Patentansprüche

1. Vorrichtung (1) zur Aufnahme eines Körperfluids, wobei die Vorrichtung (1) umfasst: eine erste gegenüber der Umgebung geschlossene Kammer (2) mit einem als Zylinder ausgebildeten zylindrischen Abschnitt und einer im Zylinder verschieblich gelagerten Wandung (4) und eine zweite gegenüber der Umgebung geschlossene Kammer (14) mit konstanter effektiver Größe, die in Bezug auf die verschiebliche Wandung (4) bewegbar ist, wobei die Vorrichtung (1) ausgestaltet ist, bei einer längsaxialen Bewegung der zweiten Kammer (14) in Bezug auf die verschiebliche Wandung (4) die erste Kammer (2) und die zweite Kammer (14) zu verbinden und bei einer Bewegung der zweiten Kammer (14) zusammen mit der verschieblichen Wandung (4) Körperfluid von der ersten Kammer (2) in die zweite Kammer (14) zu überführen, wobei die zweite Kammer (14) gleitverschieblich in Richtung der Längsachse der Vorrichtung zur ersten Kammer (2) in einer Führung (13) aufgenommen ist, wobei die Führung (13) eine Führungsfläche umfasst, die als Mantelfläche eines Zylinders ausgebildet ist, die konzentrisch zur Mantelfläche des zylindrischen Abschnitts der ersten Kammer ausgerichtet ist und sich im Wesentlichen parallel zur Längsachse erstreckt und ein Blockiermittel (18) vorgesehen ist, das die Relativbewegung von zweiter Kammer (14) zur Führung (13) verhindert, wobei zwischen der zweiten Kammer (14) und der Führungsfläche ein Dichtmittel in Umfangsrichtung angeordnet ist, wobei die verschiebliche Wandung (4) mit der Führung (13) verbunden ist, **dadurch gekennzeichnet dass** die Wandung (4) zur Innenfläche des Zylinders ein Abdichtelement in Form eines O-Rings aufweist.

2. Vorrichtung (1) nach Anspruch 1, mit einer ersten Kammer (2) zur Aufnahme eines Körperfluids und einer zweiten Kammer (14), **dadurch gekennzeichnet, dass** die Vorrichtung ausgestaltet ist, bei Zentrifugation zur Separation von Phasen des Körperfluids die erste und die zweite Kammer (2, 14) zu verbinden und das Körperfluid bei der Zentrifugation mittels der dabei wirkenden Kräfte aus der ersten Kammer (2) in die zweite Kammer (14) überführt wird.

3. Vorrichtung (1) nach Anspruch 1 oder 2, mit einer ersten Kammer (2) zur Aufnahme eines Körperfluids und einer zweiten Kammer (14), **dadurch gekennzeichnet, dass** die Vorrichtung ausgestaltet ist, eine längsverschiebliche Bewegung auszuführen, bei der die Vorrichtung zusammengedrückt wird und Körperfluid von der ersten Kammer (2) in die zweite Kammer (14) überführt wird.

4. Vorrichtung (1) nach einem der Ansprüche 1 bis 3, **dadurch gekennzeichnet, dass** die zwei Kammern (2, 14) nach Aufnahme des Körperfluides gegenüber der Umgebung geschlossen sind.

5. Vorrichtung (1) nach einem der Ansprüche 1 bis 4, **dadurch gekennzeichnet, dass** eine Barriere (9) zwischen der ersten Kammer (2) und der zweiten Kammer (14) angeordnet ist, welches bei einer Befüllung der ersten Kammer (2) geschlossen und beim Zentrifugieren geöffnet ist.

6. Vorrichtung (1) nach einem der Ansprüche 1 bis 5, **dadurch gekennzeichnet, dass** ein Öffner (22) zwischen erster und zweiter Kammer (2; 14) angeordnet ist, um einen Verschluss der zweiten Kammer (14) bei einer Bewegung der zweiten Kammer (14) zu öffnen.

7. Vorrichtung (1) nach einem der Ansprüche 1 bis 6, **dadurch gekennzeichnet, dass** ein Selektionsmittel (12) zwischen der ersten und zweiten Kammer (2, 14) angeordnet ist.

8. Vorrichtung (1) nach einem der Ansprüche 1 bis 7, **dadurch gekennzeichnet, dass** ein Septum (7) zum Wiederverschließen der ersten Kammer (2) nach einer Befüllung vorhanden ist.

9. Vorrichtung (1) nach einem der Ansprüche 1 bis 8, **dadurch gekennzeichnet, dass** eine die innere Oberfläche der ersten Kammer (2) vergrößernde Substanz (21) in der ersten Kammer (2) angeordnet ist.

10. Vorrichtung (1) nach einem der Ansprüche 1 bis 9, **dadurch gekennzeichnet, dass** die zweite Kammer (14) einen Unterdruck oder einen Luftauslass aufweist.

11. Vorrichtung (1) nach einem der Ansprüche 1 bis 10, **dadurch gekennzeichnet, dass** die Bewegung der verschieblichen Wandung (4) mittels eines Anschlagelementes begrenzt ist.

12. Vorrichtung (1) nach einem der Ansprüche 1 bis 11, **dadurch gekennzeichnet, dass** die Führung (13) als beweglicher Kolben ausgestaltet ist.

## Claims

1. Device (1) for accommodating a body fluid wherein the device (1) comprises:
A first chamber (2) closed from the environment with a cylindrical section designed as a cylinder and a wall (4) which can move relocatably in the cylinder and a second chamber (14) closed from the environment with a constant effective size which is movable in relation to the relocatable wall (4) wherein the device (1) is constructed to connect the first chamber (2) and the second chamber (14) due to a longitudinal axial movement of the second chamber (14) in relation to the relocatable wall (4) and, when moving the second chamber (14) together with the relocatable wall (4), to transfer body fluid from the first chamber (2) into the second chamber (14), wherein the second chamber (14) is slidingly relocatable along the longitudinal axis of the device to the first chamber (2) in a guide (13) wherein the guide (13) comprises a guide surface which is designed as the shell surface of a cylinder which is aligned concentrically to the shell surface of the cylindrical section of the first chamber and extends essentially parallel to the longitudinal axis, wherein a locking means (18) is provided which prevents the relative movement of the second chamber (14) to the guide (13) wherein, between the second chamber (14) and the guide surface a sealing means is arranged in the circumferential direction, wherein the relocatable wall (4) is connected to the guide (13), **characterised in that** the wall (4) has a sealing element in the form of an O-ring against the inner surface of the cylinder.

2. Device (1) according to claim 1, with a first chamber (2) to accommodate a body fluid and a second chamber (14), **characterised in that** the device is designed to connect the first and second chambers (2, 14) during centrifugation to separate phases of the body fluid, and the body fluid is transferred from the first chamber (2) into the second chamber (14) during the centrifugation by means of the forces acting on them.

3. Device (1) according to claims 1 or 2, with a first chamber (2) to accommodate a body fluid and a second chamber (14), **characterised in that** the device is designed to perform a longitudinal relocating movement during which the device is compressed, and body fluid is transferred from the first chamber (2) into the second chamber (14).

4. Device (1) according to any one of claims 1 to 3, **characterised in that**, after accommodating the body fluid, the two chambers (2, 14) are closed from the environment.

5. Device (1) according to any one of claims 1 to 4, **characterised in that** a barrier (9) is arranged between the first chamber (2) and the second chamber (14) which is closed when filling the first chamber (2) and is opened during centrifuging.

6. Device (1) according to any one of claims 1 to 5, **characterised in that** an opener (22) is arranged between first and second chambers (2; 14) in order to open a seal of the second chamber (14) during a movement of the second chamber (14).

7. Device (1) according to any one of claims 1 to 6, **characterised in that** a selection means (12) is arranged between the first and second chambers (2, 14).

8. Device (1) according to any one of claims 1 to 7, **characterised in that** a septum (7) is present to reclose the first chamber (2) after filling.

9. Device (1) according to any one of claims 1 to 8, **characterised in that** a substance (21) increasing the inner surface of the first chamber (2) is arranged in the first chamber (2).

10. Device (1) according to any one of claims 1 to 9, **characterised in that** the second chamber (14) has a vacuum or an air outlet.

11. Device (1) according to any one of claims 1 to 10, **characterised in that** the movement of the relocatable wall (4) is restricted by means of a stop element.

12. Device (1) according to any one of claims 1 to 11, **characterised in that** the guide (13) is designed as a movable piston.

## Revendications

1. Dispositif (1) destiné à contenir un fluide de corps, dispositif (1) comprenant :
une première chambre fermée à l'air ambiant (2) avec une section cylindrique formée comme un cylindre et une paroi (4) montée coulissante dans le cylindre et une deuxième chambre fermée à l'air ambiant (14) de taille constante effective, qui est mobile par rapport à la paroi coulissante (4), le dispositif (1) étant conçu pour relier la première chambre (2) à la deuxième chambre (14) lors d'un mouvement axial longitudinal de la deuxième chambre (14) par rapport à la paroi coulissante (4) et pour transférer le fluide de corps de la première chambre (2) dans la deuxième chambre (14) lors d'un mouvement de la deuxième chambre (14) avec la paroi coulissante (4), la deuxième chambre (14) étant logée mobile dans un guide (13) dans le sens de l'axe longitudinal du dispositif vers la première chambre (2), le guide (13) comprenant une surface de guidage, qui est formée comme une surface enveloppante d'un cylindre, qui est alignée de manière concentrique sur la surface enveloppante de la section cylindrique de la première chambre et s'étend essentiellement parallèlement à l'axe longitudinal et est prévue avec un élément de blocage (18), qui empêche le mouvement relatif de la deuxième chambre (14) vers le guide (13), un moyen d'étanchéité étant disposé sur le pourtour entre la deuxième chambre (14) et la surface de guidage, la paroi coulissante (4) étant reliée au guide (13), **caractérisé en ce que** la paroi (4) par rapport à la surface intérieure du cylindre présente un élément d'étanchéité sous la forme d'un anneau torique.

2. Dispositif (1) selon la revendication 1 avec une première chambre (2) destinée à contenir un fluide de corps et une deuxième chambre (14), **caractérisé en ce que** le dispositif est conçu pour relier la première chambre à la deuxième (2, 14) par centrifugation pour la séparation de phases du fluide de corps et pour transférer le fluide de corps de la première chambre (2) dans la deuxième chambre (14) par centrifugation à l'aide des forces agissant.

3. Dispositif (1) selon la revendication 1 ou 2 avec une première chambre (2) destinée à contenir un fluide de corps et une deuxième chambre (14), **caractérisé en ce que** le dispositif est conçu pour effectuer un mouvement coulissant longitudinal, lors duquel le dispositif est comprimé et le fluide de corps est transféré de la première chambre (2) dans la deuxième chambre (14).

4. Dispositif (1) selon l'une des revendications 1 à 3, **caractérisé en ce que** les deux chambres (2, 14) sont fermées à l'air ambiant une fois contenant le fluide de corps.

5. Dispositif (1) selon l'une des revendications 1 à 4, **caractérisé en ce qu'**une barrière (9) est disposée entre la première chambre (2) et la deuxième chambre (14),
barrière qui est fermée lors d'un remplissage de la première chambre (2) et ouverte lors de la centrifugation.

6. Dispositif (1) selon l'une des revendications 1 à 5, **caractérisé en ce qu'**un ouvreur (22) est disposé entre la première et la deuxième chambres (2 ; 14) afin d'ouvrir un verrouillage de la deuxième chambre (14) par un mouvement de la deuxième chambre (14).

7. Dispositif (1) selon l'une des revendications 1 à 6, **caractérisé en ce qu'**un moyen de sélection (12) est disposé entre la première et la deuxième chambres (2, 14).

8. Dispositif (1) selon l'une des revendications 1 à 7, **caractérisé en ce qu'**un septum (7) est présent pour refermer la première chambre (2) après un remplissage.

9. Dispositif (1) selon l'une des revendications 1 à 8, **caractérisé en ce qu'**une substance grossissante (21) la surface de la première chambre (2) est disposée dans la première chambre (2).

10. Dispositif (1) selon l'une des revendications 1 à 9, **caractérisé en ce que** la deuxième chambre (14) présente une sous-pression ou une sortie d'air.

11. Dispositif (1) selon l'une des revendications 1 à 10, **caractérisé en ce que** le mouvement de la paroi coulissante (4) est limité par un élément de butée.

12. Dispositif (1) selon l'une des revendications 1 à 11, **caractérisé en ce que** le guide (13) est conçu comme un piston mobile.
